(19) **Europäisches Patentamt**
**European Patent Office**
**Office européen des brevets**

(11) **EP 4 078 172 B1**

(12) **EUROPÄISCHE PATENTSCHRIFT**

(45) Veröffentlichungstag und Bekanntmachung des Hinweises auf die Patenterteilung:
**12.03.2025   Patentblatt 2025/11**

(21) Anmeldenummer: **20839014.6**

(22) Anmeldetag: **21.12.2020**

(51) Internationale Patentklassifikation (IPC):
***G01N 33/00*** *(2006.01)*

(52) Gemeinsame Patentklassifikation (CPC):
**G01N 33/0006;** G01N 33/006

(86) Internationale Anmeldenummer:
**PCT/EP2020/087501**

(87) Internationale Veröffentlichungsnummer:
**WO 2021/123443 (24.06.2021 Gazette 2021/25)**

(54) **VERFAHREN UND VORRICHTUNG ZUM BETREIBEN EINES GASSENSORS**

METHOD AND DEVICE FOR OPERATING A GAS SENSOR

PROCÉDÉ ET DISPOSITIF DE FONCTIONNEMENT D'UN CAPTEUR DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **20.12.2019   DE 102019220455**

(43) Veröffentlichungstag der Anmeldung:
**26.10.2022   Patentblatt 2022/43**

(73) Patentinhaber: **Robert Bosch GmbH**
**70442 Stuttgart (DE)**

(72) Erfinder:
• **SAUTER, Ulrich**
**76199 Karlsruhe (DE)**
• **HANAUER, Matthias Martin**
**71229 Leonberg (DE)**
• **FALKNER, Stefan**
**71272 Renningen (DE)**
• **HILDEBRAND, Felix Eberhard**
**70176 Stuttgart-West (DE)**

(56) Entgegenhaltungen:
WO-A1-2019/128203     CN-A- 106 405 007
CN-A- 109 472 303

EP 4 078 172 B1

**Beschreibung**

Technisches Gebiet

Die Erfindung betrifft Gassensoren, und insbesondere Verfahren zum Bestimmen eines Konzentrationswertes unter Berücksichtigung von lagerungsbedingten Veränderungen der Empfindlichkeit von Gassensoren.

Technischer Hintergrund

Gassensoren sind zur Bestimmung von Konzentrationen von gasförmigen Stoffen bekannt und können grundsätzlich als chemische Sensoren mit verschiedenen Sensorprinzipien und sensitiven Materialien ausgebildet sein. In der Regel führt dabei eine chemische Reaktion oder Absorption einer zu messenden gasförmigen Komponente an einer sensitiven Schicht im Sensoraufbau zu einer Eigenschaftsänderung der sensitiven Schicht. Diese Eigenschaftsänderung der sensitiven Schicht kann als eine Änderung einer elektrischen Größe detektiert und ausgewertet werden.

Je nach verwendetem Sensormaterial ist das Verhalten des Gassensors mehr oder weniger stabil, und so kann sich der Zustand des Gassensors während der Lebensdauer abhängig von Umgebungsbedingungen ändern. Insbesondere während Lagerzeiten können sich die Sensoreigenschaften während einer Lagerungsdauer durch Einflüsse wie Umgebungstemperatur, Luftfeuchtigkeit, Kontaminationen der Umgebungsluft und dergleichen derart verändern, dass eine zuverlässige und genaue Bestimmung einer Gaskonzentration einer Gaskomponente in einem Messgas nicht mehr möglich ist. Dieses Problem tritt vor allem bei Gassensoren auf, bei denen organische Materialien und insbesondere organische Halbleiter in der sensitiven Schicht verwendet werden, da in der sensitiven Schicht signalverändernde Moleküle während Lagerzeiten aufgenommen oder abgegeben werden können.

Die Druckschrift CN106405 007 B offenbart ein Kalibrierungsverfahren für einen Gassensor mit den Schritten: Aufstellen eines Sensorkalibrierungsmodells, wobei eine Prüfkammer Kalibrierungsdaten für das Kalibrierungsmodell liefert und die linearen und nichtlinearen Parameter der Einflussfaktoren der verschiedenen Sensoren in dem Kalibrierungsmodell anhand der von der Prüfkammer gelieferten Standardkalibrierungsdaten bestimmt. Das Kalibrierungsmodell basiert auf einem künstlichen neuronalen Netz, das automatisch die Temperatur und die Feuchtigkeit des Sensors aus den experimentellen Daten der experimentellen Daten extrahieren kann.

Die Druckschrift WO 2019/128203 A1 offenbart einen Netzwerkmonitor für toxische und schädliche Gase mit einer Sensordetektionskomponente, umfassend: ein Gehäuse, eine Hauptsteuerplatine in dem Gehäuse und ein Detektionsmodul, ein Datenverarbeitungsmodul und ein Stromversorgungssystem, die jeweils mit der Hauptsteuerplatine verbunden sind. Das Detektionsmodul umfasst zwei oder mehr Sensoren für toxische und schädliche Gase, um eine Echtzeitdetektion einer Vielzahl von toxischen und schädlichen Gasen zu realisieren; die Hauptsteuerplatine ist mit einer Signalerfassungseinheit, einer drahtlosen Übertragungseinheit, einer GPS-Positionierungseinheit und einer Datenspeichereinheit versehen. Die Hauptsteuerkarte ist über eine drahtlose Übertragungseinheit mit dem auf dem Internet der Dinge basierenden Managementsystem verbunden, um die Erkennungsdaten über das Internet der Dinge an das Managementsystem zu übertragen.

Die Druckschrift CN 109 472 303 A1 offenbart ein Gassensor-Driftkompensationsverfahren auf der Grundlage einer selbstkodierenden Netzwerkentscheidung, mit folgenden Schritten: Auswählen von eindimensionalen oder mehrdimensionalen unterschiedlichen Eigenwerten in den Gassensor-Array-Daten und Erhalten von entsprechenden Mittelwerten, und Auswählen und Entfernen der anormalen Abtastpunkte des Sensor-Arrays, deren entsprechende Eigenwerte größer oder kleiner als ein bestimmtes Vielfaches des Mittelwertes sind; Konstruieren eines Stapel-Selbstcodierungsnetzwerks, wobei das Stapel-Selbstcodierungsnetzwerk aus einem Rauschreduktions-Selbstcodierer und einem gemeinsamen Selbstcodierer besteht, und die vorverarbeiteten Trainingsproben-Merkmalswerte durch einen Rauschreduktions-Selbstcodierer reduziert werden und die codierten Merkmale erhalten werden, und Kombinieren der gespeicherten Merkmalswerte und der ursprünglichen Tags, um die Trainingsproben zu kodieren, und Klassifizieren der Proben durch den CART-Algorithmus, wobei die Klassifizierungen vor und nach der Probenkodierung verglichen und analysiert werden.

Offenbarung der Erfindung

Erfindungsgemäß sind ein Verfahren zum Betreiben eines Gassensors gemäß Anspruch 1 sowie eine Vorrichtung und ein Gassensor gemäß den nebengeordneten Ansprüchen vorgesehen.

Weitere Ausgestaltungen sind in den abhängigen Ansprüchen angegeben.

Gemäß einem ersten Aspekt ist ein Verfahren zum Betreiben eines Gassensorsystems mit einem Gassensor mit einer sensitiven Schicht vorgesehen, um eine Konzentrationsgröße einer Gaskonzentration einer Gaskomponente in einem Messgas bereitzustellen, mit folgenden Schritten:

- Durchführen einer Messung der Gaskonzentration während eines Messvorgangs, um einen von der Gaskonzentration abhängigen zeitlichen Verlauf eines Sensorsignals zu erhalten;
- Bestimmen der Konzentrationsgröße mithilfe eines datenbasierten Sensormodells abhängig von dem zeitlichen Verlauf des Sensorsignals, wobei das datenbasierte Sensormodell ein Sensorverhalten außerhalb des Messvorgangs zum Ermitteln der korrigierten Konzentrationsgröße berücksichtigt.

Das datenbasierte Sensormodell ist ausgebildet, um abhängig von dem zeitlichen Verlauf des Sensorsignals und abhängig von dem Sensorverhalten außerhalb des Messvorgangs die Konzentrationsgröße zu bestimmen.

Das obige Verfahren sieht vor, abhängig von einer Beobachtung eines zeitabhängigen Sensorsignals während eines Messvorgangs mit einer Beaufschlagung einer sensitiven Schicht mit Messgas und abhängig von einem Sensorverhalten außerhalb des Zeitraums des Messvorgangs mithilfe eines datenbasierten Sensormodells eine Gaskonzentration einer zu detektierenden Gaskomponente in dem Messgas in Form einer Konzentrationsgröße zu ermitteln. Die Auswertung des Sensorverhaltens außerhalb des Zeitraums des Messvorgangs ermöglicht es, den lagerungs- bzw. alterungsbedingten Sensorzustand bei der Ermittlung der Gaskonzentration zu berücksichtigen.

Insbesondere wird bei dem obigen Verfahren angenommen, dass das Sensorverhalten zeitlich außerhalb des eigentlichen Messvorgangs allgemein für den Sensorzustand charakteristisch ist. Da der eigentliche Sensorzustand in der Regel nicht direkt bestimmbar bzw. quantifizierbar ist, kann der Sensorzustand abhängig von dem Sensorverhalten mithilfe des trainierbaren datenbasierten Sensormodells bei der Ermittlung der Gaskonzentration der zu detektierenden Gaskomponenten implizit berücksichtigt werden. So kann beispielsweise das Verhalten des Sensorsignals während Aufheiz- und Abkühlphasen, die dem Zeitraum des Messvorgangs vorangehen bzw. nachfolgen, Rückschlüsse auf den lagerungs- bzw. alterungsbedingten Sensorzustand ermöglichen.

Somit kann ein Sensormodell erstellt oder bereitgestellt werden, das basierend auf den Sensorsignalen während der Messung und basierend auf messvorgangsunabhängigen Sensorsignalen bzw. Betriebsparametern des Gassensors zu einer Bestimmung einer genaueren Konzentrationsangabe der zu detektierenden Gaskomponente in dem Messgas beiträgt.

Als Betriebsparameter können insbesondere gerätespezifische Kalibrierungsparameter sowie Sensorzustände, wie eine Sensortemperatur, eine Messgastemperatur und eine Messfeuchtigkeit, berücksichtigt werden. Ein gerätespezifischer Kalibrierungsparameter kann allgemein eine geräte- bzw. sensorspezifische Information, insbesondere die Sensitivität des Gassensors, angeben.

Zudem können diese Betriebsparameter auch als Zeitreihen berücksichtigt werden. So können beispielsweise der Verlauf der Gastemperatur des Messgases, der Verlauf der Gasfeuchtigkeit des Messgases sowie der Verlauf und die Feuchtigkeit eines Spülgases, mit dem der Gassensor außerhalb der Messvorgänge durchspült wird, berücksichtigt werden.

Als datenbasiertes Sensormodell können beliebige Regressionsalgorithmen, wie z. B. Lasso, Random Forest, Gauß-Prozess und neuronale Netze, verwendet werden. Insbesondere sind bevorzugt datenbasierte Modelle, die translationsinvariant gegenüber der zeitlichen Dauer der individuellen Zeiträume, wie der Zeitraum des Messvorgangs, einer vorangehenden oder nachfolgenden Aufheiz-, Abkühl- und Haltephase, je nach Sensorbetriebsart im Messablauf sind, wie dies z. B. bei Convolutional Neural Networks der Fall ist.

Insbesondere kann das datenbasierte Sensormodell trainiert sein, um ein durch eine Alterung oder Degradation des Gassensors abhängiges Sensorverhalten zum Ermitteln der Konzentrationsgröße zu berücksichtigen.

Durch das obige Verfahren ist es möglich, die lagerungs- und alterungsbedingten Änderungen von Sensorzuständen in verbesserter Weise bei der Bestimmung einer Gaskonzentration zu berücksichtigen. Dies erfolgt durch Auswerten des Verhaltens des Gassensors, das von dem lagerungs- und alterungsbedingten Sensorzustand abhängt, insbesondere außerhalb des Zeitraums des Messvorgangs, so dass stets eine präzise Auswertung des während des Messvorgangs erhaltenen Sensorsignals möglich ist.

Das obige Verfahren stellt eine signifikante Verbesserung bezüglich herkömmlicher Kalibrierungsverfahren dar, da diese lediglich statisch sind und sich über die Zeit ändernde Sensorzustände nicht ausreichend berücksichtigen können. Zudem sind herkömmliche Kalibrierungsverfahren aufwendig und können in der Regel nicht am Einsatzort durchgeführt werden.

Insbesondere kann die korrigierte Konzentrationsgröße abhängig von einer physikalisch modellierten Konzentrationsgröße und abhängig von einer aus dem Sensormodell ermittelten Konzentrationsgröße ermittelt werden, insbesondere gemäß einer vorgegebenen Gewichtungsfunktion.

Es kann vorgesehen sein, dass das datenbasierte Sensormodell mit dem Messvorgang in Verbindung stehende Messungsgrößen und daraus abgeleitete Messungsmerkmale als Eingangsgrößen erhält.

Insbesondere können die Messungsgrößen eine oder mehrere Größen umfassen, die sich aus dem zeitabhängigen Sensorsignal während der Zeitdauer des Messvorgangs ergeben, insbesondere Werte des Sensorsignals an mehreren Abtastzeitpunkten zwischen dem Beginn und dem Ende des Messvorgangs und/oder eine oder mehrere daraus abgeleitete Größen, insbesondere eine absolute Signaländerung des Sensorsignals zwischen Beginn und Ende des Messvorgangs und/oder die maximale oder durchschnittliche Steigung des Sensorsignals S während des Messvorgangs.

Weiterhin können die Messungsgrößen eine oder mehrere Größen umfassen, die sich aus dem zeitlichen Sensorsignal während eines Zeitraums außerhalb des Zeitraums des Messvorgangs ergeben, insbesondere während einer Zeitdauer, die der Beaufschlagungsphase vorausgeht und/oder nachfolgt, insbesondere während eines Zeitraums, während dem ein bestimmter Sensorzustand eingestellt wird, insbesondere Werte des Sensorsignals an mehreren Abtastzeitpunkten zwischen dem Beginn und dem Ende des betreffenden Zeitraums und/oder einer oder mehrere daraus abgeleitete Größen, insbesondere eine absolute Signaländerung zwischen Beginn und Ende des betreffenden Zeitraums und/oder die maximale oder durchschnittliche Steigung des Sensorsignals S während des betreffenden Zeitraums.

Es kann vorgesehen sein, dass die Messungsgrößen eine oder mehrere der folgenden Größen umfassen:

- ein oder mehrere geräte- bzw. sensorspezifische Kalibrierungsparameter, die insbesondere eine Sensitivität des Gassensors angeben;
- eine oder mehrere Größen, die sich aus einem zeitlichen Signal eines oder mehrerer weiterer im System integrierter Sensoren, insbesondere einem Messgastemperatursensor, einem Messgas-Feuchtigkeitssensor sowie einem Gassensortemperatursensor, während des Zeitraums des Messvorgangs und/oder eines Zeitraums außerhalb des Zeitraums des Messvorgangs ergeben, insbesondere Werte des entsprechenden Signals an mehreren Abtastzeitpunkten zwischen dem Beginn und dem Ende des Messvorgangs oder des betreffenden Zeitraums und/oder einer oder mehrere daraus abgeleitete Größen, insbesondere eine absolute Signaländerung zwischen Beginn und Ende des betreffenden Zeitraums und/oder die maximale oder durchschnittliche Steigung des Signals des einen oder der mehreren weiter im System integrierten Sensoren während des betreffenden Zeitraums; und
- eine oder mehrere Größen, die sich aus der Differenz des zeitabhängigen Signals eines oder mehrerer im System integrierter Sensoren, insbesondere dem Gassensor, während des Zeitraums des Messvorgangs und/oder eines Zeitraums außerhalb des Zeitraums des Messvorgangs und des zeitabhängigen Signals desselben/derselben Sensoren im selben Zeitraum einer vorangegangenen Messung, insbesondere einer Kalibrierungsmessung, ergeben, insbesondere Werte der entsprechenden Differenz an mehreren Abtastzeitpunkten zwischen dem Beginn und dem Ende des Messvorgangs oder des betreffenden Zeitraums und/oder einer oder mehrere daraus abgeleitete Größen.

Weiterhin können die Messungsmerkmale aus den Messungsgrößen abgeleitet werden und eine oder mehrere der folgenden Größen umfassen:

- eine Angabe zu einer Signalantwort durch Anlegen eines Testspannungspulses,
- einen Proportionalitätsfaktor zwischen der Temperatur und dem Sensorsignal und/oder einer Zeitkonstante der Signalantwort;
- eine oder mehrere Signalantworten, die durch sprunghafte Wechsel der Zusammensetzung und/oder des Drucks des auf die Sensorfläche (31) treffenden Messgases hervorgerufen werden;
- ein Baseline-Wert, der einem Gassensor-Rohsignal zu einem definierten Zeitpunkt, insbesondere zum Zeitpunkt des Beginns des Messvorgangs, entspricht;
- einen oder mehrere Parameter physikalischer Modelle, welche an die Messdaten gefittet werden
- einen Parameter einer Exponentialfunktion, die an das Sensorsignal während der Aufheiz- oder Abkühlphase gefittet wird;
- eine oder mehrere modifizierte Messungsgrößen, insbesondere durch Kompensation des Temperatureinflusses auf das Sensorsignal mittels eines entsprechenden Proportionalitätsfaktors;
- einen oder mehrere diskrete Werte des Sensorsignals in Zeiträumen vor oder nach dem Messvorgang als Funktion der Temperatur; und
- eine Differenz oder ein Quotient zwischen nacheinander erfassten Werten mindestens einer der Messungsgrößen, insbesondere eine Differenz oder ein Quotient zwischen nacheinander erfassten Werten mindestens einer der Messungsgrößenzwischen einem aktuellen Wert der mindestens einen Messungsgröße und einem Referenzwert der mindestens einen Messungsgröße.

Gemäß einem weiteren Aspekt ist eine Vorrichtung zum Betreiben eines Gassensorsystems mit einem Gassensor vorgesehen, um eine Konzentrationsgröße einer Gaskonzentration einer Gaskomponente in einem Messgas bereitzustellen, wobei die Vorrichtung ausgebildet ist zum:

- Durchführen einer Messung der Gaskonzentration während eines Messvorgangs, um einen von der Gaskonzentration abhängigen zeitlichen Verlauf eines Sensorsignals zu erhalten; und
- Bestimmen der Konzentrationsgröße mithilfe eines datenbasierten Sensormodells abhängig von dem zeitlichen Verlauf des Sensorsignals, wobei das datenbasierte Sensormodell trainiert ist, um ein Sensorverhalten außerhalb des Messvorgangs zum Ermitteln der Konzentrationsgröße zu berücksichtigen.

Das datenbasierte Sensormodell ist ausgebildet, um abhängig von dem zeitlichen Verlauf des Sensorsignals und abhängig von dem Sensorverhalten außerhalb des Messvorgangs die Konzentrationsgröße zu bestimmen.

Gemäß einem weiteren Aspekt ist ein Gassensorsystem mit einem Gassensor und der obigen Vorrichtung vorgesehen.

Kurzbeschreibung der Zeichnungen

Ausführungsformen werden nachfolgend anhand der beigefügten Zeichnungen näher beschrieben. Es zeigen:

Figur 1     eine schematische Darstellung eines Gassensors;

Figur 2     ein Blockdiagramm zur Veranschaulichung einer Funktion zur Bestimmung einer Gaskonzentration mithilfe eines Gassensors gemäß einem Beispiel; und

Figur 3     ein Funktionsschaltbild zur Bestimmung einer Gaskonzentration mithilfe eines Gassensors gemäß einer weiteren Ausführungsform.

Beschreibung von Ausführungsformen

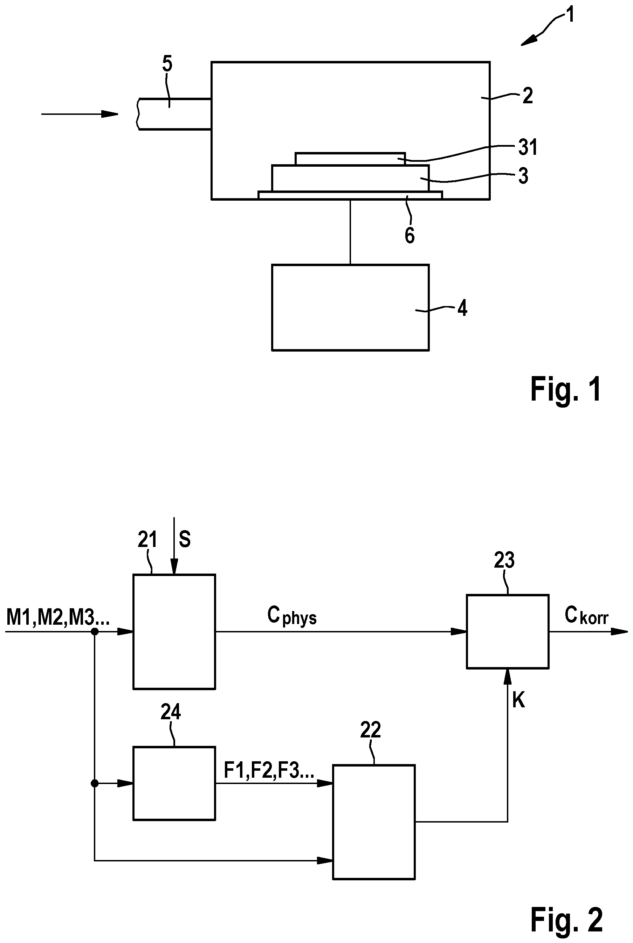

Figur 1 zeigt eine schematische Darstellung eines Gassensorsystems 1 mit einer Messkammer 2, in der ein Gassensor 3 angeordnet ist. Der Gassensor 3 weist eine Sensorfläche 31 mit einer sensitiven Schicht auf, die mit der zu detektierenden Gaskomponente beaufschlagt wird und die eine elektrische Sensorgröße bereitstellt, die eine Gaskonzentration der zu detektierenden Gaskomponente angibt.

Gesteuert durch eine Steuereinheit 4 kann in die Messkammer 2 über eine Gaszuleitung 5 ein Messgas eingebracht werden, in dem eine Konzentration einer zu detektierenden Gaskomponente gemessen werden soll. Weiterhin kann in die Messkammer 2 ein Spülgas eingebracht werden, um eine Kalibrierung des Gassensors 3 zu erreichen. In der Regel wird das Messgas über einen weiteren Kanal ausgeleitet, so dass der Gassensor 3 kontinuierlich mit dem Messgas überströmt wird.

Die Steuereinheit 4 kann lokal vorgesehen sein oder Cloud-basiert implementiert sein und in Kommunikationsverbindung mit dem Gassensor 3 stehen.

Der Gassensor 3 ist mit einer Heizeinrichtung 6 versehen, um den Gassensor 3 auf verschiedene Temperaturniveaus aufzuheizen.

Gassensoren können unterschiedliche Messprinzipien aufweisen. Insbesondere Halbleiter-Gassensoren können in einem temperaturzyklischen Betrieb betrieben werden, wobei an der Sensorfläche Sauerstoff bei einer Hochtemperaturphase angelagert wird. Der Sauerstoff wird anschließend durch die zu detektierende Gaskomponente während einer Niedrigtemperaturphase verdrängt. Die Leitfähigkeitsänderung in der Niedrigtemperaturphase kann dann entsprechend ausgewertet werden, um eine Gaskonzentration zu erhalten. Alternativ kann die Gaskonzentration des zu detektierenden Gaskomponente im Messgas auch durch die Leitfähigkeit des Halbleiter-Gassensors in einem Gleichgewichtszustand der Sensoroberfläche des Gassensors 3 ermittelt werden.

Auch andere Messprinzipien von Gassensoren sehen zeitliche Phasen mit unterschiedlichen Temperaturbeaufschlagungen vor.

Der Gassensor 3 ist allgemein so ausgebildet, dass eine chemische Reaktion, Adsorption oder Absorption einer zu messenden detektierenden Gaskomponente an der Sensorfläche 31 zu einer Eigenschaftsänderung der Sensorfläche 31 führt, die sich in einer Änderung einer elektrischen Sensorgröße (Sensorsignal) widerspiegelt, wie beispielsweise in Form einer Änderung eines Sensorstroms, einer Sensorspannung oder eines Sensorwiderstands (Sensorleitfähigkeit).

Der Gassensor 3 unterliegt während seiner Lebensdauer aufgrund von äußeren Einflüssen, wie Temperatur, Luftfeuchtigkeit und stofflichen Beaufschlagungen, einer Zustandsänderung, die den Sensorzustand und damit die Sensorempfindlichkeit verändert. Dadurch kommt es zu einer lagerungs- und alterungsbedingten Fehlkalibrierung der Gassensoren, die ausgeglichen werden sollte. Herkömmliche Verfahren sehen Nachkalibrierungen vor, die aufwändig sind und nur zu bestimmten Zeitpunkten durchgeführt werden, zwischen denen für den Gassensor 3 keine ordnungsgemäße Kalibrierung vorliegt.

Grundsätzlich soll mithilfe des Gassensorsystems 1 eine Konzentrationsgröße bereitgestellt werden. Die Konzentrationsgröße gibt die Gaskonzentration einer zu detektierenden Gaskomponente in einem die Sensorfläche 31 überspülendes Messgas an. Dabei sollen die Alterungseinflüsse und Umgebungseinflüsse eines Gassensors 3 berücksichtigt werden. Zur Ermittlung dieser Einflüsse auf die Konzentrationsmessung aufgrund einer Änderung eines lagerungs- und alterungsbedingten Sensorzustands wird in der Steuereinheit 4 ein datenbasiertes Sensormodell verwendet, trainiert ist, um

- entweder eine Korrekturgröße zur Beaufschlagung der von der gemessenen Sensorgröße abgeleiteten Konzentrationsgröße zu bestimmen, oder
- die Konzentrationsgröße direkt aus dem Verlauf der elektrischen Sensorgröße während einer Konzentrationsmessung und weiteren Betriebsparametern und/oder Verhaltensparametern des Gassensors 3 zu bestimmen .

In Figur 2 ist als Beispiel ein Funktionsdiagramm zur Veranschaulichung einer Ermittlung der Konzentrationsgröße für die Gaskonzentration einer Gaskomponente basierend auf einer durch das datenbasierte Sensormodell ermittelten Korrekturgröße gezeigt. Dazu wird mithilfe eines physikalischen Sensormodells 21, das basierend auf physikalischen Gesetzmäßigkeiten erstellt ist und das von der spezifischen Sensortechnologie abhängt, eine Roh-Konzentrationsgröße $C_{phys}$ bestimmt, die eine Gaskonzentration für einen unveränderten (z.B. nicht gealterten oder im Fertigungszustand befindlichen) Gassensor 3 angibt. Im Prinzip kann das zugrundeliegende physikalische Sensormodell 21 die Sensorempfindlichkeit bei einem neuen, nicht gealterten Gassensor 3 angeben.

Das physikalische Sensormodell 21 kann beispielsweise eine beliebige mathematische Funktion, wie beispielsweise eine Reihenentwicklung, die typischerweise eine größere Anzahl von Modellparametern aufweist, oder dergleichen, umfassen. Die Modellparameter des physikalischen Sensormodells sind dabei in der Regel global optimiert und gerätespezifisch kalibriert und somit für den individuellen Gassensor 3 fest vorgegeben.

Das Roh-Konzentrationsgröße $C_{phys}$ kann in dem Sensormodell 21 als eine Funktion aus dem Sensorsignal S und einer oder mehrerer der folgenden Messungsgrößen M1, M2, M3,... bestimmt werden:

- Größen, die sich aus dem zeitabhängigen Sensorsignal S während der Zeitdauer des Messvorgangs, d.h. während der Beaufschlagungsphase mit dem Messgas, ergeben, wie z.B. Werte des Sensorsignals an mehreren Abtastzeitpunkten zwischen dem Beginn und dem Ende des Messvorgangs und/oder daraus abgeleitete Größen, wie z. B. die absolute Signaländerung zwischen Beginn und Ende des Messvorgangs oder die maximale oder durchschnittliche Steigung des Sensorsignals S während des Messvorgangs.

- Größen, die sich aus dem zeitlichen Sensorsignal während eines Zeitraums außerhalb des Zeitraums des Messvorgangs ergeben, insbesondere während einer Zeitdauer, die der Beaufschlagungsphase vorausgeht und/oder nachfolgt, und insbesondere während eines Zeitraums, während dem ein bestimmter Sensorzustand eingestellt wird, insbesondere während eines Zeitraums eines vorbestimmten Temperaturverlaufs, insbesondere einer Aufheiz-, Abkühl- oder Temperaturhaltephase, wie z.B. Werte des Sensorsignals an mehreren Abtastzeitpunkten zwischen dem Beginn und dem Ende des betreffenden Zeitraums und/oder daraus abgeleitete Größen, wie z. B. die absolute Signaländerung zwischen Beginn und Ende des betreffenden Zeitraums oder die maximale oder durchschnittliche Steigung des Sensorsignals S während des betreffenden Zeitraums;

- ein oder mehrere gerätespezifische Kalibrierungsparameter, die zur Genauigkeit des Modells beitragen, indem sie eine gerätespezifische Information, insbesondere die Sensitivität des Gassensors, abhängig von dessen Typ zur Verfügung stellen. Diese Kalibrierungsparameter können vorgegeben und/oder empirisch bestimmt sein.

- Größen, die sich aus einem zeitlichen Signal eines oder mehrerer weiter im System integrierten Sensoren, wie beispielsweise einem Messgastemperatursensor, einem Messgas-Feuchtigkeitssensor sowie einem Gassensortemperatursensor, während des Zeitraums des Messvorgangs und/oder eines Zeitraums außerhalb des Zeitraums des Messvorgangs ergeben, wie z.B. Werte des Sensorsignals an mehreren Abtastzeitpunkten zwischen dem Beginn und dem Ende des Messvorgangs oder des betreffenden Zeitraums und/oder einer oder mehrere daraus abgeleitete Größen, insbesondere eine absolute Signaländerung zwischen Beginn und Ende des betreffenden Zeitraums und/oder die maximale oder durchschnittliche Steigung des Signals des einen oder der mehreren weiter im System integrierten Sensoren während des betreffenden Zeitraums.

- eine oder mehrere Größen, die sich aus der Differenz des zeitabhängigen Signals eines oder mehrerer im System integrierter Sensoren, insbesondere des Sensorsignals S, während des Zeitraums des Messvorgangs und/oder eines Zeitraums außerhalb des Zeitraums des Messvorgangs und des zeitabhängigen Signals desselben/derselben Sensoren im selben Zeitraum einer vorangegangenen Messung, insbesondere einer Kalibrierungsmessung, ergeben, insbesondere Werte der entsprechenden Differenz an mehreren Abtastzeitpunkten zwischen dem Beginn und dem Ende des Messvorgangs oder des betreffenden Zeitraums und/oder einer oder mehrere daraus abgeleitete Größen.

Beispielsweise kann die Roh-Konzentrationsgröße $C_{phys}$ anhand eines der folgenden mit den Modellparametern a,b,c,... parametrierten Modelle aus den so ermittelten Messgrößen M1, M2, M3,... berechnet werden mit:

- einem linearen Modell gemäß

$$C_{phys} = aM1 + bM2 + cM3 + d$$

- einem Potenzmodell gemäß

$$C_{phys} = aM1^b + cM2^d + eM3^f + \cdots + h$$

oder

- einem physikalischen Modell unter Annahme von Langmuir-Adsorption und Vernachlässigung von Desorptionsprozessen während des Messvorgangs gemäß

$$C_{phys} = f(M1, M2, M3 ..., a, b, c ...)$$

In einem Sensormodellblock 22 wird nun eine Korrekturgröße K ermittelt, mit der die aus dem physikalisch motivierten Sensormodell 21 ermittelte Roh-Konzentrationsgröße $C_{phys}$ in einem Korrekturblock 23 beaufschlagt wird, um eine korrigierte Konzentrationsgröße $C_{korr}$ zu erhalten. Insbesondere kann die Korrekturgröße K ein Korrekturfaktor zur multiplikativen Beaufschlagung der Roh-Konzentrationsgröße $C_{phys}$ oder ein Korrektur-Offset zur additiven Beaufschlagung der Roh-Konzentrationsgröße $C_{phys}$ sein.

Die Korrekturgröße K kann durch ein datenbasiertes Sensormodell in dem Sensormodellblock 22 ermittelt werden. Das datenbasierte Sensormodell kann ein Regressionsmodell, wie z. B. ein Lasso-Modell, ein Random-Forest-Modell, ein Gauß-Prozess-Modell oder ein neuronales Netz umfassen.

Als Eingangsgrößen für das Sensormodell können ausgewählte der Messungsgrößen M1, M2, M3 .....und daraus abgeleitete Messungsmerkmale F1, F2, F3... verwendet werden.

Das datenbasierte Sensormodell ermittelt die Korrekturgröße K aus einer oder mehreren der obigen Messungsgrößen M1, M2, M3 ....

Die Messungsmerkmale F1, F2, F3... können in dem Messungsmerkmalblock 24 aus den Messungsgrößen M1, M2, M3 abgeleitet werden und dienen dazu, die in den Messungsgrößen enthaltenen Information zu verdichten. Beispielsweise können die Messungsmerkmale F1, F2, F3... eine oder mehrere der folgenden Messungsmerkmale umfassen:

- eine Signalantwort durch Anlegen eines Testspannungspulses,
- einen Proportionalitätsfaktor zwischen der Temperatur und dem Sensorsignal und/oder einer Zeitkonstante der Signalantwort;
- Signalantworten, die durch sprunghafte Wechsel der Zusammensetzung (insbesondere Feuchtigkeit) und/oder des Drucks des auf die Sensorfläche 31 treffenden Messgases hervorgerufen werden. Diese Wechsel treten insbesondere zu Beginn oder Ende des Messvorgangs bei einem Wechsel der Gaszusammensetzung auf;
- Ein Baseline-Wert, der einem Gassensor-Rohsignal zu einem definierten Zeitpunkt (z.B. zum Zeitpunkt des Beginns des Messvorgangs) entspricht;
- Einen oder mehrere Parameter physikalischer Modelle, welche an die Messdaten gefittet werden. Beispielsweise kann ein Modell der Adsorptions- und Desorptionskinetik an das Sensorsignal während der Beaufschlagungs- und ggfs. nachfolgender Haltephase gefittet werden, und die gefitteten physikalischen Parameter (z.B. Adsorptionsenthalpie, Ratenkonstante) als Messungsmerkmal für das Sensormodell verwendet werden.
- Einen Parameter empirischer Zusammenhänge, welche an die Messdaten gefittet werden, wie z.B. ein Parameter einer Exponentialfunktion, die an das Sensorsignal während der Aufheiz- oder Abkühlphase gefittet wird.
- Modifizierte Messungsgrößen, insbesondere durch Kompensation des Temperatureinflusses auf das Gassensor-Rohsignal mittels eines entsprechenden Proportionalitätsfaktors;
- Einen oder mehrere diskrete Werte des Sensorsignals in Zeiträumen vor oder nach dem Messvorgang als Funktion der Temperatur
- Umgerechnete Sensorwerte von relativer auf absolute Gasfeuchtigkeit anhand der Antoine-Gleichung
- eine Differenz oder ein Quotient zwischen nacheinander erfassten Werten mindestens einer der Messungsgrößen mindestens einer der Messungsgrößen, insbesondere eine Differenz oder ein Quotient zwischen nacheinander erfassten Werten mindestens einer der Messungsgrößen zwischen einem aktuellen Wert der mindestens einen Messungsgröße und einem Referenzwert der mindestens einen Messungsgröße.

Das datenbasierte Sensormodell wird mit Trainingsdaten trainiert, die Messungsmerkmale einer zugeordneten Konzentrationsgröße (ground truth) zuordnen. Die Messungsmerkmale erhält man aus Messungsgrößen von jeweils mit einem Kalibrierungsmessgas beaufschlagten Gassensor 3. Die Konzentrationsgröße entspricht jeweils der Gaskonzentration in dem Kalibrierungsmessgas, wobei die Gassensoren 3 unter verschiedenen Lagerbedingungen gelagert worden sind. Durch Rückrechnen basierend auf der Konzentrationsgröße, die man von dem physikalisch motivierten Modell erhält, kann das datenbasierte Sensormodell in entsprechender Weise korreliert werden.

Zum Training des datenbasierten Sensormodells können mithilfe einer Variation von Lagerhistorien, d. h. zeitlichen Abfolgen von Umgebungsbedingungen z. B. durch kontrollierte Variation der Lagerungsdauer, Temperatur und Luftfeuchtigkeit, und unter Verwendung von Kalibrierungsmessgasen mit bekannter Konzentration der zu detektierenden Gaskomponente experimentell gelabelte Paare aus Messungsgrößen und Messungsmerkmalen und Gaskonzentration erzeugt werden.

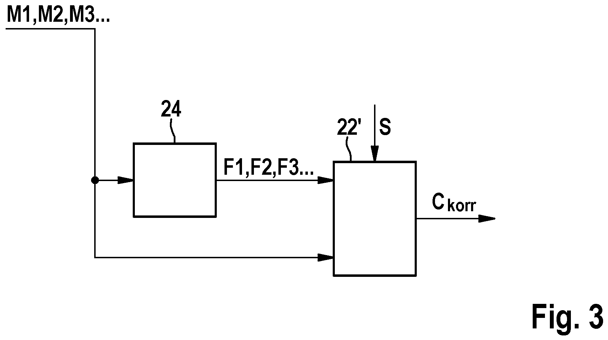

Gemäß einer Ausführungsform, wie sie in dem Funktionsdiagramm der Figur 3 gezeigt ist, kann das datenbasierte Sensormodell in einem Sensormodellblock 22' unmittelbar die korrigierte Konzentrationsgröße $C_{korr}$ ermitteln. Dabei wird das physikalische Sensormodell zusammen mit der von dem jeweiligen Sensorzustand abhängigen Korrektur in dem datenbasierten Sensormodell implementiert, um die korrigierte Konzentrationsgröße $C_{korr}$ als Modellausgang zu ermitteln.

In einem hybriden Modellansatz können das physikalische Sensormodell und das datenbasierte Sensormodell parallel korrigierte Konzentrationsgrößen bestimmen. Mithilfe einer nachgeordneten Gewichtungsfunktion können beide korrigierten Konzentrationsgrößen zueinander gewichtet werden, wobei die Gewichtung von den jeweiligen korrigierten Konzentrationsgrößen abhängig gewählt sein kann, insbesondere gemäß einer vorgegebenen Gewichtungsfunktion.

**Patentansprüche**

1. Verfahren zum Betreiben eines Gassensorsystems (1) mit einem Gassensor (3) mit einer sensitiven Schicht, um eine Konzentrationsgröße einer Gaskonzentration einer Gaskomponente in einem Messgas bereitzustellen, mit folgenden Schritten:

   - Durchführen einer Messung der Gaskonzentration während eines Messvorgangs, um einen von der Gaskonzentration abhängigen zeitlichen Verlauf eines Sensorsignals (S) zu erhalten;
   - Bestimmen der Konzentrationsgröße ($C_{korr}$) mithilfe eines datenbasierten Sensormodells abhängig von dem zeitlichen Verlauf des Sensorsignals (S), wobei das datenbasierte Sensormodell trainiert ist, um ein Sensorverhalten außerhalb des Messvorgangs zum Ermitteln der Konzentrationsgröße ($C_{korr}$) zu berücksichtigen,
   - wobei das datenbasierte Sensormodell ausgebildet ist, um abhängig von dem zeitlichen Verlauf des Sensorsignals (S) und abhängig von dem Sensorverhalten außerhalb des Messvorgangs die Konzentrationsgröße ($C_{korr}$) zu bestimmen.

2. Verfahren nach Anspruch 1, wobei das datenbasierte Sensormodell ein Gaußprozessmodell, einen LASSO-Algorithmus, einen Random-Forest-Algorithmus oder ein neuronales Netz umfasst, wobei das datenbasierte Sensormodell trainiert ist, um ein Sensorverhalten zum Ermitteln der Konzentrationsgröße ($C_{korr}$) zu berücksichtigen.

3. Verfahren nach Anspruch 1 oder 2, wobei die Konzentrationsgröße ($C_{korr}$) abhängig von einer physikalisch modellierten Konzentrationsgröße ($C_{phys}$) und abhängig von einer mithilfe des Sensormodells ermittelten Konzentrationsgröße ($C_{korr}$) ermittelt wird, insbesondere gemäß einer vorgegebenen Gewichtungsfunktion.

4. Verfahren nach einem der Ansprüche 1 bis 3, wobei das datenbasierte Sensormodell mit dem Messvorgang in Verbindung stehende Messungsgrößen und/oder daraus abgeleitete Messungsmerkmale (F1, F2, F3...) als Eingangsgrößen erhält.

5. Verfahren nach Anspruch 4, wobei die Messungsgrößen (M1, M2, M3 .....) eine oder mehrere der folgenden Größen umfassen:

   - Größen, die sich aus dem zeitabhängigen Sensorsignal (S) während der Zeitdauer des Messvorgangs ergeben, insbesondere Werte des Sensorsignals (S) an mehreren Abtastzeitpunkten zwischen dem Beginn und dem Ende des Messvorgangs und/oder eine oder mehrere daraus abgeleitete Größen, insbesondere eine absolute Signaländerung des Sensorsignals (S) zwischen Beginn und Ende des Messvorgangs und/oder die maximale oder durchschnittliche Steigung des Sensorsignals (S) während des Messvorgangs.

6. Verfahren nach Anspruch 5, wobei die Messungsgrößen (M1, M2, M3 .....) eine oder mehrere der folgenden Größen umfassen:

   - Größen, die sich aus dem zeitlichen Sensorsignal (S) während eines Zeitraums außerhalb des Zeitraums des Messvorgangs ergeben, insbesondere während einer Zeitdauer, die der Beaufschlagungsphase vorausgeht und/oder nachfolgt, insbesondere während eines Zeitraums, während dem ein bestimmter Sensorzustand eingestellt wird, insbesondere Werte des Sensorsignals (S) an mehreren Abtastzeitpunkten zwischen dem Beginn und dem Ende des betreffenden Zeitraums und/oder einer oder mehrere daraus abgeleitete Größen, insbesondere eine absolute Signaländerung zwischen Beginn und Ende des betreffenden Zeitraums und/oder die maximale oder durchschnittliche Steigung des Sensorsignals (S) während des betreffenden Zeitraums.

7. Verfahren nach Anspruch 5 oder 6, wobei die Messungsgrößen (M1, M2, M3 .....) eine oder mehrere der folgenden Größen umfassen:

   - ein oder mehrere gerätespezifische Kalibrierungsparameter, die insbesondere eine Sensitivität des Gassensors (3) angeben; und/oder
   - eine oder mehrere Größen, die sich aus einem zeitlichen Signal eines oder mehrerer weiterer im System integrierten Sensoren, insbesondere einem Messgastemperatursensor, einem Messgas-Feuchtigkeitssensor sowie einem Gassensortemperatursensor, während des Zeitraums des Messvorgangs und/oder eines Zeitraums außerhalb des Zeitraums des Messvorgangs ergeben, insbesondere Werte des entsprechenden Signals an mehreren Abtastzeitpunkten zwischen dem Beginn und dem Ende des Messvorgangs oder des betreffenden Zeitraums und/oder einer oder mehrere daraus abgeleitete Größen, insbesondere eine absolute Signaländerung zwischen Beginn und Ende des betreffenden Zeitraums und/oder die maximale oder durchschnittliche Steigung des Signals des einen oder der mehreren weiter im System integrierten Sensoren während des betreffenden Zeitraums.

8. Verfahren nach einem der Ansprüche 5 bis 7, wobei die Messungsmerkmale (F1, F2, F3...) aus den Messungsgrößen (M1, M2, M3 .....) abgeleitet werden und eine oder mehrere der folgenden Größen umfassen:

- eine Angabe zu einer Signalantwort durch Anlegen eines Testspannungspulses,
- einen Proportionalitätsfaktor zwischen der Temperatur und dem Sensorsignal (S) und/oder einer Zeitkonstante der Signalantwort;
- eine oder mehrere Signalantworten, die durch sprunghafte Wechsel der Zusammensetzung und/oder des Drucks des auf die Sensorfläche (31) treffenden Messgases hervorgerufen werden;
- ein Baseline-Wert, der einem Gassensor-Rohsignal zu einem definierten Zeitpunkt, insbesondere zum Zeitpunkt des Beginns des Messvorgangs, entspricht;
- einen oder mehrere Parameter physikalischer Modelle, welche an die Messdaten gefittet werden
- einen Parameter einer Exponentialfunktion, die an das Sensorsignal (S) während der Aufheiz- oder Abkühlphase gefittet wird;
- eine oder mehrere modifizierte Messungsgrößen (M1, M2, M3 .....), insbesondere durch Kompensation des Temperatureinflusses auf das Sensorsignal (S) mittels eines entsprechenden Proportionalitätsfaktors;
- einen oder mehrere diskrete Werte des Sensorsignals in Zeiträumen vor oder nach dem Messvorgang als Funktion der Temperatur; und
- eine Differenz oder ein Quotient zwischen nacheinander erfassten Werten mindestens einer der Messungsgrößen (M1, M2, M3 .....), insbesondere eine Differenz oder ein Quotient zwischen nacheinander erfassten Werten mindestens einer der Messungsgrößen (M1, M2, M3 .....) zwischen einem aktuellen Wert der mindestens einen Messungsgröße (M1, M2, M3 .....) und einem Referenzwert der mindestens einen Messungsgröße (M1, M2, M3 .....).

9. Vorrichtung zum Betreiben eines Gassensorsystems (1) mit einem Gassensor (3) mit einer sensitiven Schicht, um eine Konzentrationsgröße ($C_{korr}$) einer Gaskonzentration einer Gaskomponente in einem Messgas bereitzustellen, wobei die Vorrichtung ausgebildet ist zum:

- Durchführen einer Messung der Gaskonzentration während eines Messvorgangs, um einen von der Gaskonzentration abhängigen zeitlichen Verlauf eines Sensorsignals (S) zu erhalten; und
- Bestimmen der Konzentrationsgröße ($C_{korr}$)

mithilfe eines datenbasierten Sensormodells abhängig von dem zeitlichen Verlauf des Sensorsignals (S), wobei das datenbasierte Sensormodell trainiert ist, um ein Sensorverhalten außerhalb des Messvorgangs zum Ermitteln der Konzentrationsgröße ($C_{korr}$) zu berücksichtigen,
- wobei das datenbasierte Sensormodell ausgebildet ist, um abhängig von dem zeitlichen Verlauf des Sensorsignals (S) und abhängig von dem Sensorverhalten außerhalb des Messvorgangs die Konzentrationsgröße ($C_{korr}$) zu bestimmen.

10. Gassensorsystem (1) mit einem Gassensor (3) und einer Vorrichtung nach Anspruch 9.

11. Computerprogramm, welches dazu eingerichtet ist, alle Schritte eines Verfahrens nach einem der Ansprüche 1 bis 8 in einer Vorrichtung gemäß Anspruch 9 auszuführen.

12. Elektronisches Speichermedium, auf welchem ein Computerprogramm nach Anspruch 11 gespeichert ist.

**Claims**

1. Method for operating a gas sensor system (1) having a gas sensor (3) with a sensitive layer in order to provide a concentration variable of a gas concentration of a gas component in a measurement gas, having the following steps of:

- measuring the gas concentration during a measurement operation in order to obtain a temporal progression of a sensor signal (S) that is dependent on the gas concentration;
- determining the concentration variable ($C_{korr}$) with the aid of a data-based sensor model on the basis of the temporal progression of the sensor signal (S), wherein the data-based sensor model is trained to take into account a sensor behaviour outside the measurement operation in order to determine the concentration variable ($C_{korr}$),
- wherein the data-based sensor model is designed to determine the concentration variable ($C_{korr}$) on the basis of the temporal progression of the sensor signal (S) and on the basis of the sensor behaviour outside the measurement operation.

2. Method according to Claim 1, wherein the data-based sensor model comprises a Gaussian process model, a LASSO algorithm, a random forest algorithm or a neural network, wherein the data-based sensor model is trained to take into account a sensor behaviour in order to determine the concentration variable ($C_{korr}$).

3. Method according to Claim 1 or 2, wherein the concentration variable ($C_{korr}$) is determined on the basis of a physically modelled concentration variable ($C_{phys}$) and on the basis of a concentration variable ($C_{korr}$) determined with the aid of the sensor model, in particular according to a predefined weighting function.

4. Method according to one of Claims 1 to 3, wherein the data-based sensor model receives measurement variables associated with the measurement operation and/or measurement features (F1, F2, F3...) derived therefrom as input variables.

5. Method according to Claim 4, wherein the measurement variables (M1, M2, M3 ...) comprise one or more of the following variables:

- variables which result from the time-dependent sensor signal (S) during the duration of the measurement operation, in particular values of the sensor signal (S) at a plurality of sampling times between the beginning and the end of the measurement operation and/or one or more variables derived therefrom, in particular an absolute signal change in the sensor signal (S) between the beginning and the end of the measurement operation and/or the maximum or average gradient of the sensor signal (S) during the measurement operation.

6. Method according to Claim 5, wherein the measurement variables (M1, M2, M3 ...) comprise one or more of the following variables:

- variables which result from the temporal sensor signal (S) during a period outside the period of the measurement operation, in particular during a duration which precedes and/or follows the application phase, in particular during a period during which a particular sensor state is set, in particular values of the sensor signal (S) at a plurality of sampling times between the beginning and the end of the relevant period and/or one or more variables derived therefrom, in particular an absolute signal change between the beginning and the end of the relevant period and/or the maximum or average gradient of the sensor signal (S) during the relevant period.

7. Method according to Claim 5 or 6, wherein the measurement variables (M1, M2, M3 .....) comprise one or more of the following variables:

- one or more device-specific calibration parameters which indicate, in particular, a sensitivity of the gas sensor (3); and/or
- one or more variables which result from a temporal signal from one or more further sensors integrated in the system, in particular a measurement gas temperature sensor, a measurement gas humidity sensor and a gas sensor temperature sensor, during the period of the measurement operation and/or a period outside the period of the measurement operation, in particular values of the corresponding signal at a plurality of sampling times between the beginning and the end of the measurement operation or of the relevant period and/or one or more variables derived therefrom, in particular an absolute signal change between the beginning and the end of the relevant period and/or the maximum or average gradient of the signal from the one or more sensors also integrated in the system during the relevant period.

8. Method according to one of Claims 5 to 7, wherein the measurement features (F1, F2, F3...) are derived from the measurement variables (M1, M2, M3 ...) and comprise one or more of the following variables:

- an indication of a signal response by virtue of the application of a test voltage pulse,
- a proportionality factor between the temperature and the sensor signal (S) and/or a time constant of the signal response;
- one or more signal responses which are caused by sudden changes in the composition and/or the pressure of the measurement gas hitting the sensor surface (31);
- a baseline value corresponding to a gas sensor raw signal at a defined time, in particular at the time of the beginning of the measurement operation;
- one or more parameters of physical models which are fitted to the measurement data;
- a parameter of an exponential function which is fitted to the sensor signal (S) during the heating or cooling phase;
- one or more modified measurement variables (M1, M2, M3 ...), in particular by virtue of compensating for the temperature influence on the sensor signal (S) by means of a corresponding proportionality factor;
- one or more discrete values of the sensor signal in periods before or after the measurement operation as a function of the temperature; and
- a difference or a quotient between successively captured values of at least one of the measurement variables (M1, M2, M3 ...), in particular a difference or a quotient between successively captured values of at least one of the measurement variables (M1, M2, M3 ...) between a current value of the at least one measurement variable (M1, M2, M3 ...) and a reference value of the at least one measurement variable (M1, M2, M3 ...).

9. Device for operating a gas sensor system (1) having a gas sensor (3) with a sensitive layer in order to provide a concentration variable ($C_{korr}$) of a gas concentration of a gas component in a measurement gas, wherein the device is designed to:

- measure the gas concentration during a measurement operation in order to obtain a temporal progression of a sensor signal (S) that is dependent on the gas concentration; and
- determine the concentration variable ($C_{korr}$) with the aid of a data-based sensor model on the basis of the temporal progression of the sensor signal (S), wherein the data-based sensor model is trained to take into account a sensor behaviour outside the measurement operation in order to determine the concentration variable ($C_{korr}$),
- wherein the data-based sensor model is designed to determine the concentration variable ($C_{korr}$) on the basis of the temporal progression of the sensor signal (S) and on the basis of the sensor behaviour outside the measurement operation.

10. Gas sensor system (1) having a gas sensor (3) and a device according to Claim 9.

11. Computer program which is configured to carry out all steps of a method according to one of Claims 1 to 8 in a device according to Claim 9.

12. Electronic storage medium on which a computer program according to Claim 11 is stored.

**Revendications**

1. Procédé pour faire fonctionner un système de détection de gaz (1), comprenant un capteur de gaz (3) pourvu d'une couche sensible, afin de fournir une grandeur de concentration d'une concentration de gaz d'un composant gazeux dans un gaz de mesure, comprenant les étapes suivantes consistant à :

- réaliser une mesure de la concentration de gaz pendant une opération de mesure afin d'obtenir une évolution dans le temps d'un signal de capteur (S) en fonction de la concentration de gaz ;
- déterminer la grandeur de concentration ($C_{korr}$)

à l'aide d'un modèle de capteur basé sur des données en fonction de l'évolution dans le temps du signal de capteur (S), le modèle de capteur basé sur des données étant entraîné pour prendre en compte un comportement du capteur en dehors de l'opération de mesure afin de déterminer la grandeur de concentration ($C_{korr}$),
- le modèle de capteur basé sur des données étant configuré pour déterminer la grandeur de concentration ($C_{korr}$) en fonction de l'évolution dans le temps du signal de capteur (S) et en fonction du comportement du capteur en dehors de l'opération de mesure.

2. Procédé selon la revendication 1, le modèle de capteur basé sur des données comprenant un modèle de processus gaussien, un algorithme LASSO, un algorithme de forêt d'arbres décisionnels ou un réseau neuronal, le modèle de capteur basé sur des données étant entraîné pour prendre en compte un comportement de capteur afin de déterminer la quantité de concentration ($C_{korr}$) .

3. Procédé selon la revendication 1 ou 2, la grandeur de concentration ($C_{korr}$) étant déterminée en fonction d'une grandeur de concentration *($C_{phys}$)* modélisée physiquement et en fonction d'une grandeur de concentration ($C_{korr}$) déterminée à l'aide du modèle de capteur, notamment selon une fonction de pondération prédéfinie.

4. Procédé selon l'une des revendications 1 à 3, le modèle de capteur basé sur des données recevant comme grandeurs d'entrée des grandeurs de mesure qui sont associées à l'opération de mesure et/ou des caractéristiques de mesure (F1, F2, F3...) qui en sont dérivées.

5. Procédé selon la revendication 4, les grandeurs de mesure (M1, M2, M3, .....) comprenant une ou plusieurs des grandeurs suivantes :

- des grandeurs qui résultent du signal de capteur (S) dépendant du temps pendant la durée de l'opération de mesure, notamment des valeurs du signal de capteur (S) à plusieurs moments d'échantillonnage entre le début et la fin de l'opération de mesure et/ou une ou plusieurs grandeurs qui en sont dérivées, notamment une variation absolue du signal de capteur (S) entre le début et la fin de l'opération de mesure et/ou la pente maximale ou moyenne du signal de capteur (S) pendant l'opération de mesure.

6. Procédé selon la revendication 5, les grandeurs de mesure (M1, M2, M3, .....) comprenant une ou plusieurs des grandeurs suivantes :

- des grandeurs qui résultent du signal de capteur (S) temporel pendant une période en dehors de la période de l'opération de mesure, notamment pendant une période qui précède et/ou suit la phase de sollicitation, notamment pendant une période pendant laquelle un état de capteur déterminé est réglé, notamment des valeurs du signal de capteur (S) à plusieurs moments d'échantillonnage entre le début et la fin de la période concernée et/ou une ou plusieurs grandeurs qui en sont dérivées, notamment une variation absolue du signal entre le début et la fin de la période concernée et/ou la pente maximale ou moyenne du signal de capteur (S) pendant la période concernée.

7. Procédé selon la revendication 5 ou 6, les grandeurs de mesure (M1, M2, M3, .....) comprenant une ou plusieurs des grandeurs suivantes :

- un ou plusieurs paramètres d'étalonnage spécifiques à l'appareil, qui indiquent notamment une sensibilité du capteur de gaz (3) ; et/ou
- une ou plusieurs grandeurs qui résultent d'un signal temporel d'un ou plusieurs capteurs supplémentaires intégrés dans le système, notamment un capteur de température de gaz de mesure, un capteur d'humidité de gaz de mesure ainsi qu'un capteur de température de capteur de gaz, pendant la période de l'opération de mesure et/ou une période en dehors de la période de l'opération de mesure, notamment des valeurs du signal correspondant à plusieurs moments d'échantillonnage entre le début et la fin de l'opération de mesure ou de la période concernée et/ou une ou plusieurs grandeurs qui en sont dérivées, notamment une variation absolue du signal entre le début et la fin de la période concernée et/ou la pente maximale ou moyenne du signal d'un ou de plusieurs capteurs supplémentaires intégrés dans le système pendant la période concernée.

8. Procédé selon l'une des revendications 5 à 7, les caractéristiques de mesure (F1, F2, F3...) étant déduites des grandeurs de mesure (M1, M2, M3 .....) et comprenant une ou plusieurs des grandeurs suivantes :

- une indication à propos d'une réponse de signal par application d'une impulsion de tension de test,
- un facteur de proportionnalité entre la température et le signal de capteur (S) et/ou une constante de temps de la réponse du signal ;
- une ou plusieurs réponses de signal qui sont provoquées par des changements brusques de la composition et/ou de la pression du gaz de mesure qui vient frapper la surface du capteur (31) ;
- une valeur de référence qui correspond à un signal brut de capteur de gaz à un moment défini, notamment au moment du début de l'opération de mesure ;
- un ou plusieurs paramètres de modèles physiques qui sont ajustés aux données de mesure ;
- un paramètre d'une fonction exponentielle qui est ajusté au signal du capteur (S) pendant la phase de chauffage ou de refroidissement ;
- une ou plusieurs grandeurs de mesure (M1, M2, M3 .....) modifiées, notamment par compensation de l'influence de la température sur le signal de capteur (S) au moyen d'un facteur de proportionnalité correspondant ;
- une ou plusieurs valeurs discrètes du signal de capteur dans les périodes avant ou après l'opération de mesure en fonction de la température ; et
- une différence ou un quotient entre des valeurs acquises successivement d'au moins l'une des grandeurs de mesure (M1, M2, M3 .....), notamment une différence ou un quotient entre des valeurs acquises successivement d'au moins l'une des grandeurs de mesure (M1, M2, M3 .....) entre une valeur actuelle de l'au moins une grandeur de mesure (M1, M2, M3 .....) et une valeur de référence de l'au moins une grandeur de mesure (M1, M2, M3 .....).

9. Dispositif pour faire fonctionner un système de détection de gaz (1), comprenant un capteur de gaz (3) pourvu d'une couche sensible, afin de fournir une grandeur de concentration ($C_{korr}$) d'une concentration de gaz d'un composant gazeux dans un gaz de mesure, le dispositif étant configuré pour :

- réaliser une mesure de la concentration de gaz pendant une opération de mesure afin d'obtenir une évolution dans le temps d'un signal de capteur (S) en fonction de la concentration de gaz ; et
- déterminer la grandeur de concentration ($C_{korr}$) à l'aide d'un modèle de capteur basé sur des données en fonction de l'évolution dans le temps du signal de capteur (S), le modèle de capteur basé sur des données étant entraîné pour prendre en compte un comportement du capteur en dehors de l'opération de mesure afin de déterminer la grandeur de concentration ($C_{korr}$),
- le modèle de capteur basé sur des données étant configuré pour déterminer la grandeur de concentration ($C_{korr}$) en fonction de l'évolution dans le temps du signal de capteur (S) et en fonction du comportement du capteur en dehors de l'opération de mesure.

**10.** Système de détection de gaz (1) comprenant un capteur de gaz (3) et un dispositif selon la revendication 9.

**11.** Programme informatique, lequel est conçu pour exécuter toutes les étapes d'un procédé selon l'une des revendications 1 à 8 dans un dispositif selon la revendication 9.

**12.** Support de stockage électronique sur lequel est stocké un programme informatique selon la revendication 11.

Fig. 1

Fig. 2

M1,M2,M3...

24

F1,F2,F3...

22'  S

C_korr

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 106405007 B
- WO 2019128203 A1
- CN 109472303 A1